Europäisches Patentamt

European Patent Office

Office européen des brevets

(19)

(11) Publication number: **0 424 931 A2**

# EUROPEAN PATENT APPLICATION

(12)

(21) Application number: 90120445.3

(22) Date of filing: 25.10.90

(51) Int. Cl.⁵: **G01N 33/569**

(30) Priority: 27.10.89 US 428258

(43) Date of publication of application:
02.05.91 Bulletin 91/18

(84) Designated Contracting States:
**BE CH DE DK ES FR GB IT LI LU NL SE**

(71) Applicant: **E.I. DU PONT DE NEMOURS AND COMPANY**
**1007 Market Street**
**Wilmington Delaware 19898(US)**

(72) Inventor: **Devash, Yair**
**2538 Raven Road**
**Wilmington, Delaware 19810(US)**
Inventor: **Reagan, Kevin James**
**21 South Cliffe Drive**
**Wilmington, Delaware 19810(US)**

(74) Representative: **von Kreisler, Alek et al**
**Patentanwälte von Kreisler Selting Werner**
**Deichmannhaus am Hauptbahnhof**
**W-5000 Köln 1(DE)**

(54) Immunoassays which select virus-specific neutralizing antibodies.

(57) This invention relates to immunochemical assay methods which select neutralizing antibodies, more specifically, virus-specific neutralizing antibodies.

EP 0 424 931 A2

# IMMUNOASSAYS WHICH SELECT VIRUS-SPECIFIC NEUTRALIZING ANTIBODIES

Field of the Invention

This invention relates to immunochemical assay methods which select neutralizing antibodies, more specifically, virus-specific neutralizing antibodies.

Background of the Invention

Protection against pathogen-caused disease is dependent upon a number of factors including avoiding exposure to the etiologic agent, effective humoral or cellular immune responses which limit the agent's pathology, and pre- or post-exposure vaccination.

Humoral immunity relates to the presence or absence, and the type and levels of antibodies in the blood. While antibodies may be an important factor in limiting viral disease, not all antibodies are protective. Many virus-specific antibodies do not diminish viral infectivity and some may even have a detrimental effect. For that reason, immunochemical assays which measure total antibody against a particular virus may be of little value in predicting immunity.

Laboratory evidence of protective humoral immunity is therefore most often assessed in bioassays which measure neutralization of live virus in cell culture. Such a virus-neutralization bioassay determines the effectiveness of a particular blood serum to inhibit the ability of a live virus to infect susceptible cells. In addition to being costly and time consuming, viral bioassays present an obvious biohazard and require special containment facilities and other precautions.

The mechanism by which antibodies neutralize viruses is not fully understood and may differ depending on whether the virus is enveloped (surrounded by a lipid membrane, as for example, influenza virus, retroviruses, and rhabdoviruses) or non-enveloped (for example, picornaviruses, adenoviruses, papovaviruses; see e.g., Dimmock, N. J., J. Gen. Virol. 65 : 1015-1022 (1984)). It is clear that, whatever the actual mechanism, neutralizing antibody binds to a site(s) on the virus structure which subsequently inhibits that virus' ability to infect susceptible cells and tissues.

It is known that the level of serum antibodies that blocks proliferation of human immunodeficiency virus type 1 (HIV-1, also HTLV-III) (neutralizing antibodies) or blocks the fusion of HIV-infected and noninfected cells in cell culture (fusion-blocking antibodies) is relatively low when compared to the overall humoral response to the virus (i.e., the total levels of HIV-specific antibodies).

A number of HIV-1 peptides and proteins have been reported to elicit neutralizing antibodies in animals. These include synthetic peptides corresponding to amino acid sequences from the gag -coded protein [Sarin et al., 1986, Science 232 : 1135-1137], the env -coded transmembrane protein (gp41) [Kennedy et al., 1987, J. Biol. Chem. 262 : 5769-5774], and several peptides corresponding to conserved regions of the env -coded gp 120 protein. [Sun et al., 1989, J. Virol. 63 : 3579-3585; Ho et al., 1988, Science 239 : 1021-1023).

Numerous recent studies have documented the importance of domains within the env -coded external envelope glycoprotein (gp120) of HTLV-III for the binding and neutralization of the virus. This information has been summarized by Javaherian, K. et al., 1989,Proc. Natl.Acad. Sci. USA 86 : 6768-6772.

Vaccine development studies have centered on the C-terminal domain of gp120 corresponding to amino acids number 287-467 of HTLV-III$_B$ (the region represented by the recombinant fragment designated PB1) as the region containing the neutralizing domain, as summarized by Rusche, J. R. et al., 1988, Proc. Natl. Acad. Sci. USA 84 : 6924-6928.

Additional studies have used synthetic peptides (i.e., a chemically synthesized string of amino acids) to map the antigenic site of gp 120 that evokes virus type-specific neutralizing responses in mammals (Rusche, J. R. et al., 1988, Proc. Natl. Acad. Sci. USA 85 : 3198-3202; Goudsmit, J. et al., 1988, Proc. Natl. Acad. Sci. USA 85 : 4478-4482). Amino acids number 301 to 324 (numbering according to Los Alamos sequence data, Human Retroviruses and AIDS , 1989, Los Alamos National Laboratory, Los Alamos, NM) have been reported to be the immunodominant domain which is a binding site of the HIV neutralizing antibodies from infected chimpanzees and humans as well as animals immunized with gp120 or the PB1 region. The principal neutralizing domain (PND) has been mapped to the loop region between the two cysteines positioned at amino acid sequences number 296 and 331.

EP 0 424 931 A2

These amino acid sequences contain a highly conserved amino acid $\beta$-turn tetramer, GPGR, with variable sequences on both sides of the $\beta$-turn (Palker, T. J. et al., 1988, Proc.Natl. Acad. Sci. USA 85 : 1932-1936; Rusche, J. R. et al., 1988, Proc. Natl. Acad. Sci. USA 85 : 3198-3202; Goudsmit, J. et al., 1988, Proc. Natl. Acad. Sci. USA 85 : 4478-4482; Skinner, M. A. et al., 1988, J. Virol. 62 : 4195-4200; Looney, D. J. et al., 1988, Science 241 : 357-359; Drummond, J. E. et al., 1988, IV International Conference on AIDS, June 12-16, Stockholm, Sweden; and Javaherian, K. et al., 1989, Proc. Natl. Acad. Sci. USA 86 : 6768-6772).

The principal neutralizing domain of the HIV-1 is reported to be contained within the cysteine-cysteine "loop" region encompassing gp 120 amino acids 296 to 331 in the HTLV-III$_B$ strain. The corresponding region in other HIV strains (HTLV-III$_{MN}$, HTLV-III$_{RF}$, HTLV-III$_{Z3}$, etc.) may easily be identified using standard computer programs (available from, e.g., IntelliGenetics, Inc., Mountain View, CA, or Genetics Computer Group, University of Wisconsin Biotechnology Center, Madison, WI) which align for amino acid sequence homology. Conserved amino acid substitutions which do not alter the structure of the principal neutralizing domain are also included. As used herein, with respect to HIV-1 strains, the term "principal neutralizing domain" includes amino acid sequences of varying length from the loop region of HIV-1 strains which elicit virus neutralizing antibodies. It has recently been reported that as few as eight amino acids, encompassing the highly conserved Gly-Pro-Gly sequence, may comprise the principal neutralizing domain. Javaherian et al., 1989, Proc. Natl. Acad. Sci. USA 86 , 6768-6772. The principal neutralizing domain may be of actual viral origin, or it may be a synthetic or recombinant peptide or protein. The principal neutralizing domain may be utilized alone or as part of a larger peptide or protein.

The immunodominant region is not necessarily synonymous with the principal neutralizing domain in HIV, other viruses, or other pathogens. For purposes of this invention, in general principal neutralizing domain means that sequence of amino acids which is recognized by naturally occuring protective antibodies, and which, when bound by such protective antibody, results in inactivation of the etiological agent. Other neutralizing domains may exist but reaction of these peptide sequences with antibodies does not result in as efficient removal of the etiological agent from the host.

Considering the variability of the amino acid residues within the region of the principal neutralizing domain (PND) reported among known HIV-1 strains or isolates, two critical issues for development of vaccines and diagnostics are i) the prevalence of distinct virus strains within various human populations, and ii) the nature of the immune response to antigens containing this region.

Studies have now demonstrated that the majority of HIV-infected individuals worldwide, and the great majority (over 90%) in Europe and United States, are infected with viruses antigenically related to HTLV-III$_{MN}$ (Devash, Y. et al., AIDS Res. and Human Retroviruses , in press). Direct analysis of principal neutralizing domain (PND) sequences from HTLV-III strains using polymerase chain reaction (PCR) have confirmed the prevalence of the HTLV-III$_{MN}$ sequence family (which includes MN, SC, WMJ1, WMJ3, SL, and related escape mutants) (Putney, S. D. et al., 1989, V International Conference on AIDS, 1989, June 4-9, Montreal, Canada). As used herein, HTLV-III$_{MN}$ includes the HTLV-III$_{MN}$ sequence family.

It has recently been noted that amino acid changes within the principal neutralizing domain of the "loop" region may result in strains which are no longer neutralized by the host. Such "escape mutants" show amino acid changes within the hypervariable region on either side of the GPGR sequence. Assays for neutralizing antibodies to such escape mutants should utilize an antigenic peptide which reflects such mutational changes.


Summary of the Invention


This invention provides a method for the immunochemical selection of neutralizing antibodies in a biological sample, comprising contacting an antigen comprising a neutralizing domain and said biological sample under conditions whereunder the antigen-antibody reaction is biased toward binding of tightly binding antibodies and against binding of weakly binding antibodies, and detecting the immunological reaction of the neutralizing antibodies and the neutralizing domain of the antigen.

Preferably, the invention relates to a method wherein a viral neutralizing domain (or domains) is employed and virus-specific neutralizing antibodies are selected. More preferably, an HIV neutralizing domain (or domains) is employed and HIV neutralizing antibodies are selected. Preferably, the HIV neutralizing domain (or domains) will include the principal neutralizing domain (PND) from the gp120 protein. It may be desirable to include in an assay antigens which correspond to the principal neutralizing domain from more than one HIV strain, preferably including a principal neutralizing domain or domains from the HTLV-III$_{MN}$ sequence family.

The invention demonstrates that the presence of HIV-neutralizing antibodies in a mother and/or neonate is an important indicator of non-infection in the neonate, so that the method of the invention may be used to determine whether or not a neonate is HIV infected.

More specifically, the invention relates to an immunoassay which measures antibodies directed toward synthetic peptides derived from an immunodominant region of HIV gp120 comprising a principal neutralizing domain, which are responsible for virus neutralization. The binding profile and intensity, generated by an antigen-limited immunoassay of the invention, reflect a measurement of different antibody populations at the different coating concentrations of antigen used. At high antigen coating levels, the majority of antibody populations are bound. This results in maximum observed cross reactivity. At low antigen coating levels, such cross reactivity is eliminated and only the tightly binding antibodies are measured. Surprisingly, the results obtained at low antigen coating levels correlate with virus neutralization patterns as determined in bioassays. The antigen-limited immunoassay, therefore, provides a superior reflection of the biological function of the antibodies than is available in current immunoassays since it eliminates antigen-antibody cross reactions which fail to correlate with HIV neutralization.

Other immunoassay conditions/reagents which affect the antigen-antibody interaction so as to eliminate weak antigen-antibody interactions may be utilized to achieve the same results.

The immunochemical methods of the invention provide substantial advantages over HIV-neutralization bioassays, which are expensive, take days to run, and utilize HIV-infected cell lines which require special laboratory facilities and technicians. These methods provide an important diagnostic and prognostic tool for monitoring disease progression in HIV infected individuals, especially in pediatric patients. Such immunochemical methods and assays are also expected to be extremely useful in monitoring neutralizing antibody response to vaccines or potential vaccines against HIV. More specifically, this invention could be used in the development process to screen for new vaccines against a variety of pathogens.

The methods of the invention may be advantageously used in the form of a kit. Such a kit will comprise a collection of materials containing the major components used for detecting neutralizing antibody according to the methods of the invention, including a container comprising the antigen comprising a neutralizing domain. For example, a kit suitable for detecting neutralizing antibodies in a biological sample from a mother or neonate may be extremely useful in a hospital laboratory setting.

## Brief Description of the Figures

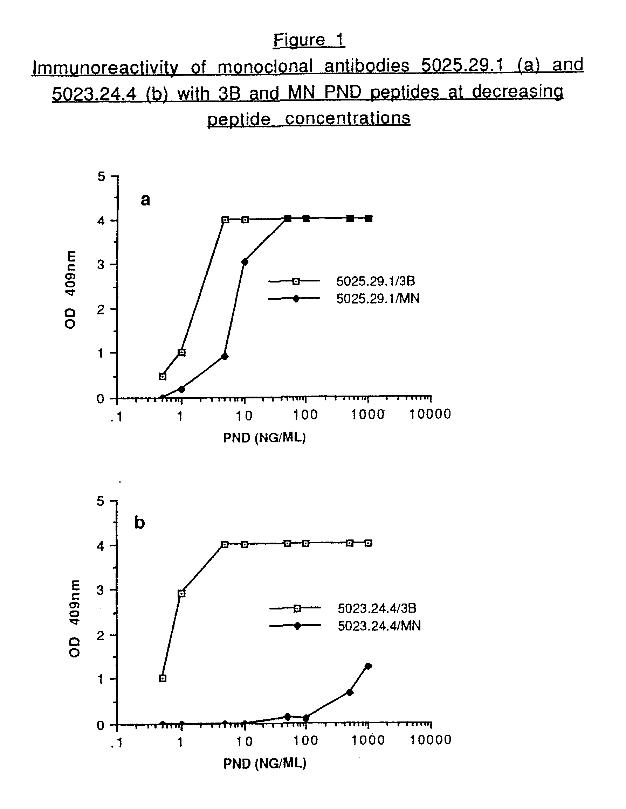

Figures 1a and 1b demonstrate the binding in immunochemical assays (antigen-limited ELISA) of monoclonal antibodies designated 5025.29.1 and 5023.24.4, respectively, to PND peptides from the HTLV-III$_B$ and HTLV-III$_{MN}$ strains. Both antibodies were raised to the HTLV-III$_B$ principal neutralizing domain (PND) peptide. Both monoclonal antibodies exhibit tight binding to the HTLV-III$_B$ peptide, whereas only the 5025.29.1 antibody also exhibits tight binding to the HTLV-III$_{MN}$ peptide. The immunochemical assay results fully correspond with viral neutralization bioassay data, and can be optimized to quantitate activity of neutralizing antibodies.

## Detailed Description of the Invention

In the studies cited above, under "Background of the Invention", antibodies were detected using either PB1 proteins or synthetic peptides from the principal neutralizing domain of gp120 bound to a solid phase. In general, the methodologies involved in development of any immunoassay emphasize the optimization of each step, so that the assay will perform in a way that maximizes activity (See e.g., Denmark, J. R. and Chessum, B. S., 1978, Med. Lab. Sci. 35 : 227; Hermann, J. E. and Collins, M. F., 1976, J. Immunol. Methods, 10 : 363).

Critical to the immunochemical methods of the invention are conditions which affect the antigen-antibody interaction, as opposed to conditions which affect primarily the system which detects the antigen-antibody interaction, i.e., the reporter system. Conditions and reagents which affect the antigen-antibody interactions and which need to be individually and empirically optimized in the course of assay design and development include antigen concentration, ionic strength, temperature, the nature and concentration of surfactants, and the nature and concentration of carrier agents such as nonspecific proteins.

For each of these reagents/conditions there is an optimal state or range that maximizes antigen-antibody binding irrespective of affinity or avidity or the "tightness" of the binding. Under such optimal conditions, the majority of the antibody specific to the antigen comprising a neutralizing domain will be bound. In order to practice the method of the invention, however, it is necessary that one or more of such conditions or reagents shall be "suboptimal" so as to eliminate weak antigen-antibody interactions. The presence of chaotropic agents, e.g., guanidine HCl, NaCl, or ammonium or sodium thiocyanate, may also disrupt weak antigen-antibody interactions. A variation in the standard ELISA protocol using this observation is described in principle by Macdonald, et al. (J. Immunol. Meth. 106 : 191-194, 1988). Alternatively, the format of the immunoassay may itself preclude weak antigen-antibody interactions, e.g., in a capture assay where the antigen is in solution instead of being bound to a solid phase. In such an assay format, the ability of an antibody to capture the labeled antigen is a reflection of higher affinity since low affinity antibodies are known to inefficiently bind to antigens in solution.

The assay could also be devised as any competitive immunoassay, as exemplified by Yorde et al. (1978., in: Proc. Int. Symp. on Enz-Labelled Immuno. of Hormones and Drugs , New York, 359-372) where the binding of an antibody to a specific principal neutralizing domain (PND) takes place in the presence of another, detectably labeled antibody (polyclonal or monoclonal). If specificity for the principal neutralizing domain (PND) exists in the challenging antibody population (i.e., the biological sample to be tested), it will displace or compete for the binding of the known detectably labeled antibody. The result will be a decrease in assay response, proportional to the concentration of specific antibody in the challenge sample. It is known that competition assays of this sort are highly dependent upon the affinity of the labeled antibody since only biological samples comprising antibody with at least the same affinity for the target antigen will be able to displace the labeled antibody. It becomes apparent then, that one could bias the assay for tightly binding antibodies by formatting the test to use a high affinity monoclonal or polyclonal antibody to the principal neutralizing domain (PND) as the labeled antibody.

As mentioned above, suboptimal antigen concentrations may be utilized to eliminate weak antigen-antibody interactions. For example, it is generally understood by those skilled in the art of assay development, that the coating of solid surfaces by antigens involves titrating the antigen for reactivity using a standard antiserum. Such titrations typically yield a sigmoidal-shaped curve of assay response. At high antigen coating concentrations, which saturate the solid phase, a maximal assay response is noted. This is seen as a "plateau", that is, a region of the titration curve which shows little change in the amount of antibody detected regardless of the change in the amount of antigen. As antigen concentrations are lowered to non-saturating or "suboptimal" levels, assay responses decrease proportionally.

In order to achieve maximal assay sensitivity (the usual goal of immunoassays), one of skill in the art would typically choose an antigen-coating concentration which represents the minimal antigen concentration which is on the upper plateau, i.e., maximum assay response. This area represents the maximal separation of specific from non-specific signal. Coating concentrations in the linear portion of the titration curve are considered the most susceptible to variation in assay response, thus involving potential reproducibility problems. Coating concentrations in the low end of the titration curve are thought to represent an insensitive assay since a large proportion of an individual's immune response would be missed. Pollack et al. (1988, Proc. Natl. Acad. Sci. USA 85 : 2298-2302) reported that an antigen-limited ELISA for the hapten, p - azophenylarsonate, selected for monoclonal antibodies which demonstrated high avidity for the antigen.

Therefore, the immunoassays known in the art which utilize the principal neutralizing domain (PND) antigen (Drummond, J. E. et al., 1988, IV International Conference on AIDS, June 12-16, Stockholm, Sweden; Devash, Y. et al., 1989, V International Conference on AIDS, June 4-9, Montreal, Canada; Rossi, P. L. et al., 1989, V International Conference on AIDS, June 4-9, Montreal, Canada; and Javaherian, K. et al., 1989, Proc. Natl. Acad. Sci. USA 86 : 6768-6772, Devash et al. AIDS Res. and Human Retroviruses , in press) contain the PND antigen at non-rate limiting concentrations determined by coating the peptide at levels at the upper end or plateau of the antigen titration curve.

The impact of this form of optimization is to maximize weak antigen-antibody interactions which in turn maximizes detection of weak immunological cross reactivity. This is exemplified by studies where an ELISA (enzyme linked immunosorbant assay) utilizing PND-coated solid phases resulted in a prediction that human, chimpanzee, and goat sera to either recombinant fragments of gp120, or antisera prepared against defined PND's, showed a high level of cross reaction among a variety of PND's (corresponding to different HIV isolates). (Drummond, J. E. et al., 1988, IV International Conference on AIDS, June 12-16, Stockholm, Sweden; and Devash Y. et al., AIDS Res. and Human Retroviruses , in press). This did not correlate with the known neutralization pattern of the same sera in bioassays which showed neutralizing activity in only a limited number of HIV strains.

This problem of immunoassay reactions not correlating with bioassay neutralization patterns is not limited to the amino acid sequence 301-324 region of gp120. Sun et al., (J. Virol 63 : 3579-3585, 1989) described an assay where synthetic peptides representing amino acids 423 to 437 (the proposed CD4-binding domain) of gp120 were used to map the binding of seven monoclonal antibodies. The antibodies (prepared against HTLV-III$_B$) reportedly shared crossreactivity by ELISA and RIPA (radioimmune precipitation assay) with diverse HTLV-III isolates including HTLV-III$_B$, HTLV-III$_{RF}$, HTLV-III$_{AL}$, HTLV-III$_{WMJ}$, HTLV-III$_{Z84}$, and HTLV-III$_{Z34}$. However, neutralization bioassays demonstrated that the monoclonal antibodies actually neutralized only HTLV-III$_B$, HTLV-III$_{RF}$, and HTLV-III$_{AL}$.

Unlike standard immunoassays, which use antigen at optimal, non-rate limiting concentrations, the immunochemical methods of the invention utilize an antigen comprising an HIV principal neutralizing domain, and/or other antigen comprising an HIV neutralizing domain, at suboptimal, rate-limiting conditions. Surprisingly, it was found that biological specimens exhibited a binding profile and intensity of reaction in this assay which correlated well with the biologically important neutralizing function of the antibodies, as determined in viral neutralization bioassays. It is demonstrated that weak immunological cross reactive reactions, which have no correlation with the biological neutralizing activity of these antibodies, are eliminated in this assay. As demonstrated in the examples, the determination of the exact suboptimal concentration or other suboptimal reagent(s) or condition(s), will be determined experimentally in the course of assay design, and will usually require that a series of assays be prepared and that the results obtained be compared with the results of a reliable bioassay.

This invention relates to immunochemical methods for the selection of tightly-binding antibody populations which correlate to neutralizing activity. For purposes of this application, "tightly binding" means antibodies which are detected by suboptimal assay conditions, e.g., rate limiting concentrations of the antigen comprising the neutralizing domain. This tight binding can be attributed to apparent higher affinity (exhibiting a $K_d$ of at least $10^{-6}$ M, preferably at least $10^{-8}$ M, and more preferably at least $10^{-9}$ M), to higher avidity (a property of the number of antigen binding sites on an antibody), or to cooperativity (enhancement of binding by another molecule). Despite general recognition of the importance of selectively measuring virus neutralizing antibodies, prior immunochemical assays were not able to provide results which correlated with virus neutralization bioassays.

In one embodiment, the method is an indirect ELISA prepared by coating a solid phase with one or more synthetic peptides derived from one or more different HIV strains, each peptide comprising an antigen including an HIV principal neutralizing domain (PND), and preferably including an amino acid sequence corresponding to a principal neutralizing domain of HTLV-III$_{MN}$. A series of assays was prepared differing only in that each peptide was coated at varying concentrations (nanomole to femtomole range). The immobilized peptide was contacted with either PND immune sera or sera of HIV-infected individuals. Bound patient or animal antibodies were detected by the addition of an enzyme labeled anti-species conjugate. The color generated was proportional to the concentration of the bound antibodies. It was found that low (suboptimal or rate limiting) antibody coating concentrations could be used to select out neutralizing antibodies so that the ELISA results correlated with the results of virus neutralization bioassays.

This method provides a highly desirable alternative to bioassays (e.g., infectivity assays utilizing human T cells and live HIV virus) which eliminates the need for special laboratory facilities and techniques, and provides notably quicker results.

The methods described herein have a variety of uses, including quantitative assessment of HIV neutralizing antibodies in a biological sample without the necessity of conducting an HIV neutralization bioassay, a tedious (two to seven day), complex, and biohazardous procedure. Furthermore, bioassays are not available for all strains of HIV or for such other pathogens as Hepatitis B virus. The existence and level of HIV neutralizing antibodies may be a valuable indicator of patient prognosis, as well as providing information useful for directing therapeutic measures. Since the presence of HIV-neutralizing antibodies in a neonate is an important indicator of non-infection, the methods of the invention may be used to determine whether or not a neonate from an HIV-infected mother is also HIV infected. The methods may also be used for routine screening of neonates, whether or not the infant has been determined to be at risk. Since it is known that certain IgG subclasses of antibodies are transferred across the placenta, the monitoring of protective antibodies can be from samples obtained from maternal, fetal, neonatal, cord, placental, or amniotic sources. A "biological sample", as used herein, is any antibody-containing fluid such as any body fluid or other solution potentially containing antibodies. Early diagnosis of infection may permit early therapeutic or other intervention in only those infants or fetuses likely to be infected.

The methods of the invention are also expected to be valuable for monitoring responses of HIV vaccine recipients, or for screening of potential vaccine candidates. (See Duffy, J. I., Vaccine Preparation Techniques , Park Ridge, N.J., Noyes Data Corp. (1980), the teaching of which is hereby incorporated by reference.) Current vaccine development efforts are concentrating on subunit vaccines, i.e., utilization of viral fragments including isolated native proteins, recombinant proteins, and/or synthetic peptides, which include one or more neutralizing domains. A quick and reliable alternative to HIV neutralization bioassays is expected to be a critical tool for vaccine development. The development of new vaccines is dependent upon the ability to screen potential vaccine candidates for the proper antigenicity, i.e., the ability to elicit the proper protective immune response. This invention provides a methodology to enable this screening to take place in a timely manner.

The methods of the invention are not limited to the determination of virus neutralizing antibodies. The development of neutralizing antibodies is critical to the survival of higher mammals against many pathogens. Hence, it is fully expected that the principles described herein are applicable to other pathogens where the development of a protective humoral response is important biologically and diagnostically. For some viruses (e.g., rubella) where individuals are resistant to reinfection once they have been infected (and survived that initial infection), it is unnecessary to test for actual levels of neutralizing antibody since any level of antibody to the structural proteins of that virus is evidence of protection. For other viruses (e.g., herpes simplex virus), infection and development of neutralizing antibodies does not connote protection from redevelopment of symptoms, since the virus can remain dormant in an individual's nerve cells, only to reappear at various times (colds, menstruation, stress, etc.). However, for viruses against which vaccines exist, evidence of neutralizing antibodies is the preferred means of documenting protection. The purpose of a vaccine is to expose previously uninfected individuals to an antigenic stimulus which triggers the immune system to develop a protective humoral and cellular response to the relevant etiologic agent. Upon exposure to that virus, the immune system will have already developed neutralizing antibodies which can inactivate the virus before it can establish any pathology. However, proving that a vaccination has been successful is often dependent upon difficult in vitro bioassays which cannot be routinely used, but are the only alternative to animal studies.

Indeed, there are documented cases of vaccine failures for Hepatitis B and rabies viruses. These unfortunate individuals could generally be shown to have developed some antibody response, but the level or quality of that response was inadequate to protect them from subsequent exposure. An immunochemical assay which reflects an immune response that is correlated with the type of immunity demonstrated by bioassays, is critical in such cases. After known exposure, e.g., to Hepatitis B, in a previously vaccinated individual, the true level of immunity could be determined, so that appropriate measures (e.g., administration of Hepatitis Immune Globulin) could be taken where necessary. It is clear that antibody titers per se are not indicative of protective, neutralizing antibodies, just as high antibody titer does not necessarily correlate with high affinity antibody. There are frequent examples of the existence of large quantities of low affinity antibody populations exhibiting high titers in ELISA.

The term "neutralizing antibody" is frequently used to connote a protective antibody response to a virus infection. It should be recognized, however, that such protective antibody-mediated responses are not limited to viruses, but are also relevant to survival against bacterial, fungal, parasitic or other microbial infections. Resistence to various neoplastic diseases is also dependent upon a functional immune system, a portion of which is the antibody response. Therefore, as used herein, the term "neutralizing antibody" is not limited to virus neutralizing antibody, but includes any antibody which provides a protective antibody-mediated response. In the context of HIV, "neutralizing antibody" includes an antibody which has a fusion-blocking effect and/or inhibits viral infectivity in infectivity assays.

The method of the invention is not limited to the detection of antibodies protective against HIV-1, but may also be used to detect antibodies protective against picornavirus, retroviruses, reovirus, togavirus, orthomyxovirus, paramyxovirus, rhabdovirus, arenavirus, coronavirus, bunyavirus, papovavirus, adenovirus, herpetovirus, hepatnavirus or poxvirus. The same methodology may be used to determine antibodies protective against a bacteria, parasite, fungus, or microbe, or against a malignant, transformed, or abnormal cell, so long as such moiety comprises a neutralizing domain which evokes antibodies having a protective effect.

Amino acid residues are represented herein by single letter nomenclature as described in Lehninger, Biochemistry, 2nd Edition , p. 72, Worth Publishers, Inc., (1975). The teaching of the invention provided herein is believed to fully enable those of skill in the art to practice the invention. Because a number of useful sources of addition information are also referred to, however, these cited sources are hereby incorporated by reference. The following Examples illustrate but do not serve as a limitation of the invention.

EXAMPLES

7

MATERIALS AND METHODS

Preparation of synthetic peptides:

The following peptides were synthesized on an Applied Biosystems (Foster City, CA) 430A peptide synthesizer using chemical and program cycles supplied by the manufacturer. A series of synthetic peptides corresponding to env -coded amino acids 307-310 (numbering according to Los Alamos sequence data) from a variety of HIV strains was used in this study. (See Table 1, below). As indicated, some sequences were identical in several different strains:

## TABLE 1
### AMINO ACID SEQUENCE

| HIV STRAIN | #307 | #319 | |
|---|---|---|---|
| SC | RSIHI | GPGR AFYA | |
| MN | KRIHI | GPGR AFYA | |
| 3B(BH10) | IRIQR | GPGR AFVT | * |
| RF | KSITK | GPGR VIYA | |
| WMJ1 | RHIHI | GPGR AFYT | |
| WMJ2 | RSLSI | GPGR AFRT | |
| WMJ3 | RRIHI | GPGR AFYT | |
| ARV2 | KSIYI | GPGR AFHT | ** |
| Z3 | QSIRI | GPGK VFYA | |
| Z6 | QSTPI | GLGQ ALYT | |
| NY5 | KGIAI | GPGR TLYA | |
| SL | TRIHI | GPGR AFYT | |
| LAVMA | RGIHF | GPGQ ALYT | |
| LAVEL | QRTPI | GLGQ SLYT | |
| CDC42 | SRVTL | GPGR VWYT | *** |

\*    Also sequence of 3B(HXB2), H9, LAVIA, BRU, BH8, HXB3, and PV22

\*\*    Also sequence of SF2

\*\*\*    Also sequence of CDC4

After synthesis, peptides were deprotected and cleaved from the supporting resin with trifluoromethane sulfonic acid and then purified by reverse phase HPLC. The peptides were then analyzed by spectral analysis, amino acid composition, and sequencing. The length of the synthetic peptides was preferentially set as a 13-mer, but other sizes either larger or somewhat smaller, would yield substantially the same results. Similarly, a larger recombinant protein or even the native protein could have been used as long as the relevant principal neutralizing domain (PND) was represented within the protein sequence and an immunoassay format used (i.e., competitive immunoassay) which was consistent with the ability to bias detection to tightly binding antibodies to the relevant principal neutralizing domain (PND).

Sera:

*Human sera:* Sera from HIV-infected individuals were determined to contain antibodies to the virus by reactivity in HIV viral lysate ELISA, western blot, and the ENV9TM (comprising env amino acids 474-479) recombinant HIV ELISA (Du Pont).

Preparation of Monoclonal antibodies:

Balb/c x C57 B1/6 F1 mice (8 weeks of age) were immunized by the method described by Fischberg, F.D.E. et al., 1986, Immunology Today 7 : 344-346. Briefly, a single intraperitoneal inoculation of 100 µg of the peptide conjugates, mixed 1:1 with complete Freund's adjuvant was given. Four weeks later, mice were bled and peptide-specific antibody responses were evaluated. The mice were rested until their antibody titers dropped. At that time the animals were boosted with 100µg of the peptide conjugate in incomplete Freund's adjuvant.

Four days later, mice were sacrificed and their splenocytes were fused with SP 2/O-Ag 14 myeloma cells by standard methods. Hybridomas specific for the immunizing peptide were selected by non-antigin limited ELISA using PND peptide antigens and western blot using HTLV-III viral lysate. It is apparent that the methods of the invention could have been used to screen hybridomas for production of tightly binding neutralizing antibodies. Hybridomas were stabilized, cloned twice by limiting dilution, and seed lots frozen. Bulk antibody was produced by ascites formation in Balb/c x C57B1/6 F1 mice using standard methods. Antibodies from ascitic fluids were purified by ammonium sulfate precipitation and protein A affinity chromatography. The method of making the monoclonal antibodies is described in greater detail in co-assigned application U.S. Serial Number 07/324,027 filed March 20, 1989, the teaching of which is hereby incorporated by reference.

ELISA assays (Antigen-limited ELISA):

Polystyrene microtiter plates (Nunc) were coated with the synthetic peptides using 100 µl/well coating solutions at concentrations varying from 1 µg to 0.5 ng/ml, followed by a blocking step with commercially available ELISA kit diluent (Du Pont). However, the nature of the solid support as well as the means of blocking sites on the solid support after peptide binding are not considered critical to the invention. Other types of solid phases, including beads, tubes, particles, etc., or differing materials, for example, polypropylene or magnetic particles, including chromium dioxide, could have been used.

Human (or other animal) antibodies were diluted to 1:20 and incubated with the peptide coated plates for 1 hour at room temperature. Unbound antibodies were removed by washing the wells six times successively with phosphate buffered saline (PBS), containing 0.5% Tween® 20. Bound antibodies were detected by adding an appropriate rabbit anti-species IgG conjugated to alkaline phosphatase (Sigma, St. Louis, MO), then reacting with the substrate paranitrophenylphosphate (PNPP) (Sigma), and reading at 405 nm wavelength on a spectrophotometer, as is known in standard ELISA methods.

The cut-off for sera was defined as the mean optical density (OD) value of 10 seronegative samples plus five values of standard deviation. A reactive serum was defined as a serum that gave OD readings above the cut-off.

Other means of constructing the ELISA method could have been employed, as known to those skilled in the art. For example, the immunoassay could have employed different enzymes conjugated to the anti-species antibody (for example, horseradish peroxidase, or biotin followed by an avidin-enzyme conjugate). The method of detecting the reaction could also have been fluorometric, chemiluminescent, or bioluminescent. The rate of signal development could also have been measured. The conjugate could also have been radiolabelled and the immunoassay devised as an ImmunoRadioMetric Assay (IRMA), as described in principle by Reagan, K. et al., (J. Virol. 48 :660-666, 1983).

Moreover, the method of the invention is not limited to the ELISA format, but is applicable to immunoassays in general, so long as some component of the assay can be adjusted to bias the assay towards the detection of the tightly binding antibody population. For example, the peptide could have been radiolabelled or biotinylated (see below) and used in a capture principle. In such an assay format, the ability of an antibody to capture the labeled antigen is a reflection of higher affinity since low affinity antibodies are known to inefficiently bind to antigens in solution.

As discussed above, the assay could also be devised as a competitive immunoassay, as exemplified by Yorde et al. (1978., in: Proc. Int. Symp. on Enz-Labelled Immuno. of Hormones and Drugs , New York, 359-372) where the binding of an antibody to a specific principal neutralizing domain (PND) takes place in the presence of a tightly binding, detectably labeled antibody (polyclonal or monoclonal).

Other formats take advantage of the differential susceptibility of antigen-antibody reactions to chaotropic agents such as salts (e.g., guanidine HCl, NaCl, ammonium thiocyanate), surfactants, temperature, or pH, in order to bias the assay for the population of tightly binding antibodies. A variation in the standard ELISA protocol using this observation is described in principle by Macdonald et al. (J. Immunol. Meth. 106 : 191-194, 1988).

Biotinylation of synthetic peptides:

The various synthetic peptides were biotinylated by standard methods (described by the manufacturer, Pierce) using the N-hydroxysuccinimide ester of biotin (NHS-L-C-Biotin), or the biotin-L-C-hydrazide, water soluble reagents with an extended spacer arm, which reacts with primary amines or carboxyl groups. The biotinylated principal neutralizing domain(PND) peptides were then applied onto the polystyrene plates and peptide coating efficiency was determined by the detection of the biotin coupled to the peptide using streptavidin conjugated to horseradish peroxidase (HRP) (Pierce).

Neutralization assays:

*Infectivity assay:* Antibodies were assayed for virus neutralization by a microtiter syncytium-inhibition assay utilizing a sensitive clone of CEM-SS cells (Nara, P. L. et al. (1987) J. Virol. 61 : 3173-3180). Briefly, 50,000 cells, to be infected with the HIV virus, were attached to poly(L-lysine)-coated microtiter wells. HTLV-III infecting stock included HTLV-III virus at a concentration of 200-300 syncytia forming units [SFU], mixed with the neutralizing antibody at twofold incremental dilutions, or with buffer. The cells were incubated with the infecting solution for 1 hour at 22° C, after which they were washed and replaced in complete RPMI 1640 (GIBCO, Grand Island, NY). Syncytia were counted on day five and compared with values for virus control wells (no antibody added). The endpoint neutralization titer was defined as the reciprocal of the antibody dilution in which 90% of the total SFU was inhibited.

*Cell fusion inhibition assay:* The cell fusion assay was done as described (Matthews, T. J. et al. (1987), Proc. Natl. Acad. Sci. USA 84 : 5424-5428). Briefly, 5000 HIV infected cells in 30 μl were seeded into microtiter wells. The cells then were incubated for 30min at 30° C, followed by addition of 30 μl containing 75,000 MOLT4 cells in RPMI media. The plates were then incubated for 24 hours at 37° C and scored for syncytia formation (cell fusion). Neutralization titer was defined as the titer in which antibodies inhibited 50% of syncytia formation.

## Example 1

Monoclonal Antibody Binding to the Principal Neutralizing Domain Peptides at Decreasing Antigen Concentration (Antigen-limited ELISA)

The reactivity of a monoclonal antibody (designated 5025.29.1, ATCC Accession #HB10041, directed toward the HTLV-III$_B$ PND peptide) was tested at 1.5 μm IgG/ml against antigens comprising a principal neutralizing domain from gp120 from diverse HTLV-III strains. When incubated at an antigen coating concentration of 1000 ng per ml, the monoclonal antibody was highly reactive, generating high OD signals (3.5-4 OD), with all of the PNDs which contain the amino acid sequence GPGRAF. However, when antigen coating concentrations were decreased to 100, 50, 10, 5, 1, and 0.5 ng per ml, a gradation of reactivity was observed (See Table 2). The HTLV-III$_B$ PND peptide was the only peptide to which the antibody bound at the lowest peptide coating level--0.5ng/ml. The monoclonal antibody failed to bind all other PNDs at the lower antigen coating levels, suggesting that the antibody has the tightest binding to the HTLV-III$_B$ PND peptide. Variations in plate coating efficiency were ruled out since all of the PND peptides showed equivalent coating efficiency as determined by binding of biotinylated PNDs to the polystyrene plates. Differences in the binding of the 5025.29.1 antibody with the various PNDs suggested the ranking of binding strength to be: 3B > SC > WMJ3 > > MN > WMJ2 > WMJ1 = SL > CDC42 > RF > Z3 = Z6 > >NY5 > LAVMA = LAVEL.

As shown in Table 2, at lower antigen coating concentrations, reactivity was observed only with antigens to which a relatively tight binding occurs (HTLV-III$_B$ at 0.5 ng/ml, and HTLV-III$_{MN}$ and HTLV-III$_{SC}$ at 5 ng/ml coating concentrations). When this antibody was tested for HIV neutralization, the HTLV-III$_B$ strain was neutralized by the monoclonal antibody at 5μg IgG/ml. The HTLV-III$_{MN}$ strain was also neutralized, but to a lesser degree, suggesting that the antibody's stronger binding to the HTLV-III$_B$ strain is correlated to a greater biological activity of the antibody. Thus, we concluded that the suboptimal concentrations of the antigens (antigen-limited ELISA) biased the assay toward the detection of the HTLV-III neutralizing antibodies.

Table 2

| Immunoreactivity of 5025.29 1 Monoclonal Antibody with PND Peptides at Decreasing Concentrations | | | | | | | |
|---|---|---|---|---|---|---|---|
| ANTIGEN | OD (405nm) | | | | | | |
| | PND COATING CONCENTRATION (ng/ml) | | | | | | |
| | 1000 | 500 | 100 | 50 | 10 | 5 | 1 | 0.5 |
| 3B | >4.2 | >4.2 | >4.2 | >4.2 | >4.2 | >4.2 | 1.0 | 0.5 |
| SC | >4.2 | >4.2 | >4.2 | >4.2 | 3.45 | 0.95 | 0.2 | 0.01 |
| MN | >4.2 | >4.2 | >4.2 | >4.2 | 2.9 | 1.1 | 0.09 | 0.01 |
| WMJI | >4.2 | >4.2 | >4.2 | 3.73 | 2.0 | 0.9 | 0.05 | 0.005 |
| WMJ3 | >4.2 | >4.2 | >4.2 | 3.5 | 3.05 | 1.2 | 0.15 | 0.01 |
| WMJ2 | >4.2 | >4.2 | >4.2 | 2.9 | 2.0 | 0.8 | 0.1 | 0.01 |
| ARV2 | >4.2 | >4.2 | 3.4 | 2.36 | 0.03 | 0.01 | 0.01 | 0.01 |
| SL | >4.2 | >4.2 | >4.2 | >4.2 | 3.01 | 1.0 | 0.1 | 0.01 |
| CDC42 | 0.6 | 0.4 | 0.01 | 0.01 | 0.01 | 0.01 | 0.01 | 0.01 |
| RF | 0.5 | 0.3 | 0.01 | 0.01 | 0.01 | 0.01 | 0.01 | 0.01 |
| Z3 | 0.2 | 0.2 | 0.01 | 0.01 | 0.01 | 0.01 | 0.01 | 0.01 |
| Z6 | 0.18 | 0.2 | 0.01 | 0.01 | 0.01 | 0.01 | 0.01 | 0.01 |
| NY5 | 0.019 | 0.01 | 0.01 | 0.01 | 0.01 | 0.01 | 0.01 | 0.01 |
| LAVMA | 0.01 | 0.01 | 0.01 | 0.01 | 0.01 | 0.01 | 0.01 | 0.01 |
| LAVEL | 0.01 | 0.01 | 0.01 | 0.01 | 0.01 | 0.01 | 0.01 | 0.01 |

The importance of antibody binding strength in virus neutralization was further demonstrated using another monoclonal antibody, designated 5023.24.4 (ATCC #HB10043). The 5023.24.4 monoclonal antibody is also directed toward the HTLV-III$_B$ PND peptide. As shown in Figure 1, both monoclonal antibodies demonstrated tight binding to the HTLV-III$_B$ PND peptide (Figure 1 a, b; binding to HTLV-III$_B$ PND peptide at 0.5 ng/ml coating concentrations). Whereas the 5025.29.1 also demonstrated tight binding to HTLV-III$_{MN}$ PND peptide (binding to PND-MN at 10 ng/ml coating concentrations), the 5023.24.4 monoclonal antibody failed to demonstrate this type of tight binding (see Figure 1b, binding to MN only at <1000 ng/ml coating concentrations). The antigen-limited ELISA binding data fully correlated with virus neutralization data in that 5023.24.4 neutralized the HTLV-III$_B$ strain in infectivity assays as well as in fusion inhibition assays, but did not neutralize the HTLV-III$_{MN}$ strain.

The 5025.29.1 monoclonal antibody which showed modest neutralization ability against the HTLV-III$_B$ strain in infectivity assays (5μg IgG/ml, as compared to 0.5 μg IgG/ml of the 5023.24.4 antibody) also had fusion inhibition activity against both the HTLV-III$_B$ strain (5μg IgG/ml) and the HTLV-III$_{MN}$ strain (50μg IgG/ml). Hence, the antigen-limited ELISA can be easily optimized to quantitate activity of HIV neutralizing antibodies.

Together these data demonstrate that: i) an ELISA method in accordance with the prior art, which does not discriminate between strong and weak antigen-antibody interactions [i.e., an ELISA which uses higher (non-rate limiting) PND coating concentrations] will yield results which fail to correlate with the biological function of the antibody population (i.e., HTLV-III neutralization ability), and ii) an assay employing antigen-limited concentrations will be biased towards selection and measurement of the tight binding antibody subpopulations, and iii) the antibodies that bind tightly to antigen are the preferred population to be measured in relation to biological activity.

Example 2

Goat PND Antisera Binding to PND Peptides at Decreasing Antigen Concentrations and Correlation to Virus Neutralization

The ability of the assay to quantitatively correlate antibody binding at low antigen concentration with virus neutralization was also demonstrated using polyclonal antibodies generated in goats against the various PND peptides. Each goat antiserum was tested against PND peptides from three different HTLV-III strains in the antigen-limited ELISA as described above.

The results are summarized in Table 3 below. The assay detected the HIV strain to which the tightest binding existed, a result which fully correlated with the antiserum's ability to neutralize virus infectivity. At high antigen coating concentrations cross reactions to the various PNDs, with no correspondence to virus neutralization, were observed.

Table 3

| Immunoreactivity of Goat Antiserum Directed toward PNDs from Different HTLV Strains | | | | | |
|---|---|---|---|---|---|
| Goat Antiserum (HTLV Strain) | Neutralization Titer* | | | Antigen-limited ELISA (Antigen (ng/ml)** | |
| | 3B | RF | MN | 3B | RF | MN |
| Goat (3B) | 128 | <4 | <4 | 10 | 1000 | 500 |
| Goat (RF) | <4 | 4500 | <4 | 1000 | 0.5 | 100 |
| Goat (MN) | <4 | <4 | 48 | 100 | 500 | 50 |

\* The reciprocal of the antibody dilution at which 90% of the total SFU (syncytia forming units) was inhibited.
\*\* Lowest antigen coating concentration which generated an OD signal above cut off.

Example 3

Binding of HIV-1 Infected Human and Chimpanzee Sera to PND Peptides at Decreasing Antigen Concentrations and Correlation to Neutralization

The ability of the antigen-limited assay to correlate antibody binding to PND at low antigen concentration with virus neutralization was also tested using human or chimpanzee sera infected with HIV-1. Each serum was tested against three PND peptides in the antigen-limited ELISA described above. The results are summarized in Table 4. Although the assay detected the HIV strain to which the tightly binding antibody population is directed, the result was not fully correlated with the ability of the sera to neutralize virus. In natural HIV infection the humoral immune system is targeted against all of the HIV epitopes, and it is established that epitopes such as the CD4 binding site (Sun, N. C. et al., (1989) J. Virol. 63 : 3579-3585; Traunecker, A. et al., (1989) Nature 339 : 68-70, and Jameson, A. R. et al., (1988) Science 240 : 1335-1339) or other envelope epitopes (Ho, D. D. et al., (1988) Science 239 : 1021-1023; and Dalgleish, A. G. et al., (1988) Virology 165 : 209-215) may elicit neutralizing antibodies. In fact, the results presented in Table 4 confirm that there are other neutralizing domains, in addition to the gp120 principal neutralizing domain utilized herein. Both the human and the chimpanzee sera showed neutralizing potency against the RF strain although neither the antigen-limited ELISA nor a high antigen concentration ELISA detected antibodies directed toward the PND of the HTLV-III$_{RF}$ strain. As additional HIV neutralizing domains are identified, an antigen-limited ELISA or other appropriately designed immunoassay including such domains will be useful to sort between antibodies which cross react with the various PNDs and the biologically functioning neutralizing antibodies.

Table 4

| Immunoreactivity and Neutralization of Human and Chimpanzee Infected with HTLV-III | | | | |
|---|---|---|---|---|
| Strain | Human | | Chimpanzee | |
| | Ag* | Neut.** | Ag* | Neut.** |
| MN | 0.5 | >256 | 5 | >64 |
| 3B | NONE*** | >128 | 1 | >256 |
| RF | NONE*** | >256 | NONE*** | >8 |

\* Lowest antigen coating concentration which generated an OD signal above cut off.
\** The reciprocal of the antibody dilution in which 90% of the total SFU was inhibited.
\*** No ELISA signal generated at any antigen coating concentration up to 1000ng.ml.

Example 4

Determination of Maternal HIV Transmission to Neonates and Disease Prognosis

It was recently reported by Rossi et al. (V International Conference on AIDS, June 4-9, 1989, Montreal, Canada) that 31% of uninfected neonates born to HTLV-III infected mothers had maternal antibodies directed toward the gp120 PND, whereas infected neonates had no such antibodies. The ELISA method used in those experiments i) utilized an antigen (env amino acids 324 to 338) which excludes the amino acids critical for neutralization, the PND ii) was not antigen-concentration limited, thus did not distinguish among binding strengths of antibody populations and therefore was of limited prognostic value, and iii) did not utilize the sequence derived from the HTLV-III$_{MN}$ strain, which is the predominant infective strain in HIV infected individuals. In contrast, the antigen-limited ELISA assay described herein is capable of discriminating between antibodies that exhibit biologically ineffectual cross reactions directed toward the PND, and those that bind to the PND strongly and are responsible for virus neutralization.

Table 5 summarizes data obtained from fifteen maternal/neonate pairs where serum obtained at birth from mother and neonate and retained in a repository was tested in the antigen-limited ELISA of the invention. All neonates from HIV-infected mothers that had tightly binding antibodies directed toward the PND remained healthy. Since it has recently been demonstrated that most of the HTLV-III infected population is infected with the HTLV-III$_{MN}$ strain, it was considered sufficient to utilize the MN PND antigen in this test. It is clear from these results that the potential exists for monitoring fetal and neonatal well being by monitoring populations of protective maternal antibodies using the methods of the invention.

EP 0 424 931 A2

Table 5

| Maternal/Neonatal HIV Transmission: Correlation to Tightly Binding PND antibodies | | |
|---|---|---|
| [Total samples-15 Maternal/neonatal pairs] | | |
| | TIGHTLY BINDING MATERNAL Ab | LOW/MODERATE BINDING MATERNAL Ab |
| HEALTHY NEONATES | 4 | 1 |
| INFECTED NEONATES | 0 | 10 |

**Claims**

1. A method for the immunochemical selection of neutralizing antibodies in a biological sample, comprising contacting an antigen comprising a neutralizing domain and said biological sample under conditions whereunder the antigen-antibody reaction is biased toward binding of tightly binding antibodies and against binding of weakly binding antibodies, and detecting the immunological reaction of the neutralizing antibodies and the neutralizing domain of the antigen.

2. A method of Claim 1, wherein the biological sample is contacted with an antigen comprising a virus neutralizing domain and virus neutralizing antibodies are detected.

3. A method of Claim 2, wherein the biological sample is contacted with an antigen comprising an HIV neutralizing domain and HIV neutralizing antibodies are detected.

4. A method of Claim 3, wherein the biological sample is contacted with an antigen comprising an HIV principal neutralizing domain from the HIV gp120 protein and HIV neutralizing antibodies are detected.

5. A method of Claim 4, wherein the biological sample is contacted with an antigen comprising a sequence corresponding to amino acids number 307 to 319 from the HIV gp120 protein, and HIV neutralizing antibodies are detected.

6. A method of Claim 4, wherein the biological sample is contacted with an antigen comprising a sequence corresponding to amino acids number 309 to 317 from the HIV gp120 protein, and HIV neutralizing antibodies are detected.

7. A method of Claim 5, wherein the biological sample is contacted with an antigen comprising an amino acid sequence derived from a strain of the HTLV-III$_{MN}$ sequence family which corresponds to amino acids number 307 to 319 from the HIV gp120 protein, and HIV neutralizing antibodies are detected.

8. A method of Claim 6, wherein the biological sample is contacted with an antigen comprising an amino acid sequence derived from a strain of the HTLV-III$_{MN}$ sequence family which corresponds to amino acids number 309 to 317 from the HIV gp120 protein, and HIV neutralizing antibodies are detected.

9. A method of Claim 4, wherein the biological sample is contacted with an antigen comprising the amino acid sequence, RIHIGPGRAFY, and HIV neutralizing antibodies are detected.

10. A method of Claim 4, wherein the biological sample is contacted with an antigen complrising the amino acid sequence, IHIGPGRAF, and HIV neutralizing antibodies are detected.

11. A method of any one of Claim 1 to 10, wherein the conditions of the assay select for antibodies which have an apparent affinity, $K_d$, of at least $10^{-6}$ M.

12. A method of any one of Claims 1 to 10, wherein the conditions of the assay select for antibodies which have an apparent affinity, $K_d$, of at least $10^{-8}$ M.

13. A method of any one of Claims 1 to 10, wherein the conditions of the assay select for antibodies which have an apparent affinity, $K_d$, of at least $10^{-9}$ M.

14. A method of Claim 2, wherein the biological sample is contacted with an antigen comprising a corresponding virus neutralizing domain from at least two different strains of the same virus.

15. A method of Claim 3, wherein the biological sample is contacted with an antigen comprising a corresponding HIV neutralizing domain from at least two different HIV strains.

16. A method of Claim 15, wherein the biological sample is contacted with an antigen comprising an HIV principal neutralizing domain from the HIV gp120 protein of at least two different HIV strains.

17. A method of Claim 16, wherein the biological sample is contacted with an antigen comprising a sequence corresponding to amino acids number 307 to 319 from the HIV gp120 protein of at least two different HIV strains.

18. A method of Claim 16, wherein the biological sample is contacted with an antigen comprising a sequence corresponding to amino acids number 309 to 317 from the HIV gp120 protein of at least two different HIV strains.

19. A method of Claim 17, wherein at least one of said sequences is derived from an HIV strain from the HTLV-III$_{MN}$ sequence family.

20. A method of Claim 18, wherein at least one of said sequences is derived from an HIV strain from the HTLV-III$_{MN}$ sequence family.

21. A method of Claim 16, wherein the biological sample is contacted with an antigen comprising the amino acid sequence, RIHIGPGRAFY.

22. A method of Claim 16, wherein the biological sample is contacted with an antigen comprising the amino acid sequence, IHIGPGRAF.

23. A method of any one of Claims 14 to 22, wherein the conditions of the assay select for antibodies which have an apparent affinity, $K_d$, of at least $10^{-6}$ M.

24. A method of any one of Claims 14 to 22, wherein the conditions of the assay select for antibodies which have an apparent affinity, $K_d$, of at least $10^{-8}$ M.

25. A method of any one of Claims 14 to 22, wherein the conditions of the assay select for antibodies which have an apparent affinity, $K_d$, of at least $10^{-9}$ M.

26. A method of Claim 3, wherein the biological sample is contacted with at least two different HIV neutralizing domains.

27. A method of Claim 26, wherein the biological sample is contacted with at least two different HIV neutralizing domains, one of which is a principal neutralizing domain from the HIV gp120 protein.

28. A method of Claim 27, wherein the biological sample is contacted with at least two different HIV neutralizing domains, one of which comprises a sequence corresponding to amino acids number 307 to 319 from the HIV gp120 protein.

29. A method of Claim 27, wherein the biological sample is contacted with at least two different HIV neutralizing domains, one of which comprises a sequence corresponding to amino acids number 309 to 317 from the HIV gp120 protein.

30. A method of Claim 28, wherein the biological sample is contacted with at least two different HIV neutralizing domains, one of which comprises an amino acid sequence derived from a strain of the HTLV-III$_{MN}$ sequence family which corresponds to amino acids number 307 to 319 from the HIV gp120 protein.

31. A method of Claim 28, wherein the biological sample is contacted with at least two different HIV neutralizing domains, one of which comprises an amino acid sequence derived from a strain of the HTLV-III$_{MN}$ sequence family which corresponds to amino acids number 309 to 317 from the HIV gp120 protein.

32. A method of Claim 27, wherein the biological sample is contacted with an antigen comprising the amino acid sequence, RIHIGPGRAFY.

33. A method of Claim 27, wherein the biological sample is contacted with an antigen comprising the amino acid sequence, IHIGPGRAF.

34. A method of any one of Claims 26 to 33, wherein the conditions of the assay select for antibodies which have an apparent affinity, $K_d$, of at least $10^{-6}$ M.

35. A method of any one of Claims 26 to 33, wherein the conditions of the assay select for antibodies which have an apparent affinity, $K_d$, of at least $10^{-8}$ M.

36. A method of any one of Claims 26 to 33, wherein the conditions of the assay select for antibodies which have an apparent affinity, $K_d$, of at least $10^{-9}$ M.

37. A method of Claim 26, wherein the HIV neutralizing domains include a CD4-binding domain and a principal neutralizing domain of the HIV gp120 protein.

38. A method of any one of Claims 1 to 10, wherein the antigen is present at a suboptimal concentration.

39. A method of Claim 11, wherein the antigen is immobilized on a solid phase at a suboptimal concentration.

40. A method of Claim 12, wherein the antigen is immobilized on a solid phase at a suboptimal concentration.

41. A method of Claim 13, wherein the antigen is immobilized on a solid phase at a suboptimal concentration.

42. A method of any one of Claims 1 to 10, wherein the antigen comprising a neutralizing domain is detectably labeled and is contacted in solution with said biological sample under conditions which permit the detectably labeled antigen to bind with tightly binding neutralizing antibodies in the biological sample, and wherein the labeled antigen-antibody complex is captures by the antigen comprising a neutralizing domain which is unlabeled and immobilized on a solid phase.

43. A method of Claim 11, wherein the antigen comprising a neutralizing domain is detectably labeled and is contacted in solution with said biological sample under conditions which permit the detectably labeled antigen to bind with tightly binding neutralizing antibodies in the biological sample, and wherein the labeled antigen-antibody complex is captured by the antigen complrising a neutralizing domain which is unlabeled and immobilized on a solid phase.

44. A method of Claim 12, wherein the antigen comprising a neutralizing domain is detectably labeled and is contacted in solution with said biological sample under conditions which permit the detectably labeled antigen to bind with tightly binding neutralizing antibodies in the biological sample, and wherein the labeled antigen-antibody complex is captured by the antigen comprising a neutralizing domain which is unlabeled and immobilized on a solid phase.

45. A method of Claim 13, wherein the antigen comprising a neutralizing domain is detectably labeled and is contacted in solution with said biological sample under conditions which permit the detectably labeled antigen to bind with tightly binding neutralizing antibodies in the biological sample, and wherein the labeled antigen-antibody complex is captured by the antigen comprising a neutralizing domain which is unlabeled and immobilized on a solid phase.

46. A method of any one of Claims 1 to 10, which is a competitive immunoassay wherein the biological sample is contacted with the antigen comprising a neutralizing domain in the presence of a tightly binding detectably labeled antibody specific to the principal neutralizing domain.

47. A method of Claim 46 wherein the tightly binding detectably labeled antibody has an apparent affinity, $K_d$, specific to the principal neutralizing domain, of at least $10^{-6}$ M.

48. A method of Claim 46 wherein the tightly binding detectably labeled antibody has an apparent affinity, $K_d$, specific to the principal neutralizing domain, of at least $10^{-8}$ M.

49. A method of Claim 46 wherein the tightly binding detectably labeled antibody has an apparent affinity, $K_d$, specific to the principal neutralizing domain, of at least $10^{-9}$ M.

50. A method of any Claims 1 to 10, wherein the antigen-antibody interaction is biased toward detection of tightly binding antibodies by the presence of suboptimal conditions in the immunoassay, said suboptimal conditions being selected from the group consisting of antigen concentration, ionic strength, temperature, and the nature and concentration of surfactants.

51. A method of Claim 50, wherein the suboptimal conditions of the assay select for antibodies which have an apparent affinity, $K_d$, of at least $10^{-6}$ M.

52. A method of Claim 50, wherein the conditions of the assay select for antibodies which have an apparent affinity, $K_d$, of at least $10^{-8}$ M.

53. A method of Claim 50, wherein the conditions of the assay select for antibodies which have an apparent affinity, $K_d$, of at least $10^{-9}$ M.

54. A method of any one of Claims 4 to 10, used to predict HIV infection in a neonate, wherein the biological sample is from the mother or the neonate, and the presence of HIV neutralizing antibodies is detected.

55. A method of Claim 54, wherein the conditions of the assay select for antibodies which have an apparent affinity, $K_d$, of at least $10^{-6}$ M.

56. A method of Claim 54, wherein the conditions of the assay select for antibodies which have an apparent affinity, $K_d$, of at least $10^{-8}$ M.

57. A method of Claim 54, wherein the conditions of the assay select for antibodies which have an apparent affinity, $K_d$, of at least $10^{-9}$ M.

58. A method of any one of Claims 1 to 10, which is used to monitor neutralizing antibodies in a biological sample from a recipient of a vaccine or vaccine candidate comprising the antigen comprising a neutralizing domain.

59. A method of Claim 58, wherein the conditions of the assay select for antibodies which have an apparent affinity, $K_d$, of at least $10^{-6}$ M.

60. A method of Claim 58, wherein the conditions of the assay select for antibodies which have an apparent affinity, $K_d$, of at least $10^{-8}$ M.

61. A method of Claim 58, wherein the conditions of the assay select for antibodies which have an apparent affinity, $K_d$, of at least $10^{-9}$ M.

62. A method of any of Claims 1 to 4, 54, or 58 which provides a quantitative assessment of neutralizing antibodies.

63. A method of Claim 1 wherein the biological sample is derived from a hybridoma cell line and tightly binding, neutralizing monoclonal antibodies are selected.

## Figure 1

## Immunoreactivity of monoclonal antibodies 5025.29.1 (a) and 5023.24.4 (b) with 3B and MN PND peptides at decreasing peptide concentrations